# EUROPEAN PATENT SPECIFICATION

(11) **EP 3 722 435 B1**
(45) Date of publication and mention of the grant of the patent: **03.09.2025**
(21) Application number: 20176019.6
(22) Date of filing: 03.04.2017
(51) Int. Cl.: C12N 15/86

(54) **FIXED-BED BIOREACTOR WITH CONSTANT-FLOW PUMP/TUBING SYSTEM**
FESTBETTBIOREAKTOR MIT KONSTANTFLUSSPUMPEN-/-ROHRLEITUNGSSYSTEM
BIORÉACTEUR À LIT FIXE COMPORTANT UN SYSTÈME DE POMPE/TUBE À DÉBIT CONSTANT

(30) Priority: 14.04.2016 US 201662322651 P
(43) Date of publication of application: 14.10.2020
(62) Divisional of application: 17782838.1
(73) Proprietor: Ferring Ventures Ltd, West Drayton UB7 7PS (GB)
(72) Inventor: LESCH, Hanna, P., 70210 Kuopio (FI); RÄSÄNEN, Eva, Kristiina, 70870 Hiltulanlahti (FI); VALONEN, Piia, Kristiina, 70100 Kuopio (FI); TUUNANEN, Tarja, Hannele, 70780 Kuopio (FI); KARNAATTU, Milla Jonna, Karoliina, 70150 Kuopio (FI); MÜLLER, Achim, 71150 Vartiala (FI); KARHINEN, Minna, Kristiina, 70500 Kuopio (FI); YLA-HERTTUALA, Seppo, 70210 Kuopio (FI)
(74) Representative: Cabinet Becker et Associés

(56) References cited:
- WO-A1-2016/048556
- US-A1- 2002 177 215
- HANNA P. LESCH ET AL: "Process Development of Adenoviral Vector Production in Fixed Bed Bioreactor: From Bench to Commercial Scale", HUMAN GENE THERAPY, vol. 26, no. 8, 1 August 2015 (2015-08-01), US, pages 560 - 571, XP055426642, ISSN: 1043-0342, DOI: 10.1089/hum.2015.081

## Description

### Related Applications

This application asserts priority to United States provisional patent filing Serial No. 62/322651, filed 14 April 2016.

### Background

We have improved commercial-scale adherent cell culture by developing an improved bioreactor which provides a ~50% increase in productivity viz prior art bioreactors, while also eliminating a source of contamination.

Commercially-available adherent cell culture bioreactors include, for example, the laboratory-scale iCELLis^{™} Nano (commercially available from Pall Corporation, Cambridge Masachusetts) and the commercial scale iCELLis^{™} 500 bioreactor, which provides a volume of up to 65 liters of cell culture medium and a cell culture substrate of medical grade polyester microfibers which provide up to 500m² growth area available to the cells. We improved the function of such commercial-scale bioreactors by first, defining the process steps in a small scale and then scaling these up into a large scale. Pall Life Science, the manufacturer of the iCELLis^{™} brand of bioreactors, was recommending re-circulation or perfusion as a feeding strategy. The feeding strategy in small (Nano^{™}) scale was tested by re-circulation, and later optimized by perfusion.

Perfusion is a process step where cells in a bioreactor are continuously feed with a fresh medium at the same time removing equal amount of spend medium, which enables the cell growth in high cell density (Vellinga et al. 2014). The perfusion rate can vary depending on the type of cell line used, the polypeptide product produced by those cells, the specific cell culture medium employed and the cell growth system used. An important aspect in the removal of the spent medium is also to remove the (possible toxic) metabolic side products from the cell culture. These side products may have a negative effect on cell viability, and further may impair the productivity of producer or host cells.

Options for perfusion include batch perfusion and fed-batch perfusion (where feeding of fresh medium is performed but no removal of spend medium is performed). The fed-batch type approach is also a re-circulation strategy, where the cell culture medium volume is only enlarged by external medium container and medium is re-circulated from a media reservoir or container to the bioreactor and back again, wherein no actual removal of spend or used medium is done.

The manufacturer of the iCELLis^{™} line of bioreactors (Pall Life Technologies) markets the iCELLis^{™} 500 as suitable for perfusion feeding. We made a very surprising finding during our first experiment with iCELLis^{™} 500: as provided by the manufacturer, the equipment is not in fact capable of perfusion, if using the standard iCELLis^{™} 500 tubing and pump system set at the slowest pump output rate available.

We deconstructed the commercially-available apparatus and found out the reasons for failure. The commercially-available apparatus is manufactured with the feed-out tube of larger interior diameter than the feed-in tube. This configuration is intedned to prevent unwanted overflow of the liquid media by ensuring that in-flow cannot be greater than out-flow. We surprisingly found that this configuration adversely impacts pump efficiency and the productivity of the cultured cells. Rather, we found the feed-out tube is best of equal or smaller interior diameter than the feed-in tube. We found that the commercially-provided ("stock") medium feed-out tube was of too large interior diameter, providing a too-large interior volume space for the media flow rate. This large interior tube volume permitted formation of undesirable air pockets and bubbles in the tubing.

In addition, the stock feed-out tube was integrated into a too-weak pump system which could not provide an adequate media flow out from the bioreactor. This may be because formation of air bubbles in turn appears to affect the correct out feed-out pump capacity. The feed-out pump works by creating negative pressure in the feed-out tube, pulling media into the feed-out tube. Unlike liquid media, however, air bubbles expand in this negative-pressure environment, absorbing much of the negative pressure energy, thus frustrating pump work.

### Brief Description

We resolved the failures inherent in the commercially-available apparatus by retrofitting the bioreactor with a bigger peristaltic pump able to provide the proper (lower) rate of fluid output, and replacing the feed-out tubing with replacement tubing having a smaller inside diameter, thus preventing formation of undesirable air bubbles in the feed-out tubing.

Another suggestion we have made for the manufacturer is to make bioreactors with smaller tubing diameter to improve the medium flow inside tubing.

We also have designed and tested a new sampling manifold configuration which eliminates the risk that a compromised aseptic filter will contaminate an entire manufacturing batch.

The invention is as defined in the appended set of claims.

It is herein disclosed an adherent cell culture bioreactor comprising a substrate for adherent cell culture and a pump able to provide a constant-rate output of cell culture media of less than about 50 mL of media per 1,800 cells per day. In a particular embodiment, the pump is able to provide a minimum constant-rate output of not more than about 16.7 mL of media per 1,800 cells per day. In yet another embodiment, the substrate provides at least about 60 m² of surface area for adherent cell culture, in particular at least about 400 m² of surface area for adherent cell culture. In yet another embodiment, the adherent cell culture bioreactor is configured to contain at least about 20 liters of cell culture media.

It is also herein disclosed a recombinant polypeptide produced by culturing adherent producer cells in the adherent cell bioreactor described in the previous paragraph. In a particular embodiment, the polypeptide comprises virus. In yet another embodiment, the virus comprises a viral capsid containing a non-viral transgene.

It is also further herein disclosed a method for culturing adherent cells on a substrate comprising:
a. obtaining the bioreactor described in the second to last paragraph, and then
b. adding to the bioreactor cell culture media and cells, and then
c. culturing the cells in adherent mode on the substrate, while circulating the cell culture media about the cells at a rate of less than about 50 mL of media per 1,800 cells per day.

In a particular embodiment of this method, the substrate provides at least about 400 m² of surface area for adherent cell culture. In another embodiment, the bioreactor is configured to contain at least about 20 liters of cell culture media.

It is further disclosed herein an adherent-cell bioreactor comprising a container containing a substrate for adherent cell culture and a pump configured to be able to provide a constant-rate output of cell culture medium at a pump output volume sufficient to maintain in said cell culture medium a parameter selected from the group consisting of: concentration of lactate in said medium of not more than about 1.58 grams / liter; concentration of glucose in said medium of between about 0.5 and 1.0 grams / liter; and concentration of glucose in said medium of not less than about 2.89 grams / liter. In a particular embodiment, the adherent cell culture bioreactor is configured to contain at least about 20 liters of cell culture media. In another embodiment, the substrate provides at least about 60 m² of surface area for adherent cell culture, in particular at least about 400 m² of surface area for adherent cell culture. In another embodiment, the pump of the adherent-cell bioreactor is configured to be able to provide a constant-rate output of cell culture medium at a pump output volume sufficient to maintain in said cell culture medium a concentration of lactate in said medium of not more than about 1.58 grams / liter. In a particular embodiment, the pump is configured to be able to provide a constant-rate output of cell culture medium at a pump output volume sufficient to maintain in said cell culture medium a concentration of glucose in said medium of between about 0.5 and 1.0 grams / liter. In yet another embodiment, the pump is configured to be able to provide a constant-rate output of cell culture medium at a pump output volume sufficient to maintain in said cell culture medium a concentration of glucose in said medium of not less than about 2.89 grams / liter.

It is also disclosed herein a recombinant polypeptide produced by culturing adherent producer cells in the adherent cell bioreactor of the previous paragraph. In a particular embodiment, the polypeptide comprises virus. In yet another embodiment, the virus comprises a viral capsid containing a non-viral transgene.

It is further disclosed a method for culturing adherent cells on a substrate comprising:
a. obtaining the bioreactor described in the second to last paragraph, and then
b. adding to the bioreactor cell culture media and cells, and then
c. culturing the cells in adherent mode on the substrate, while circulating the cell culture media about the cells at a rate to maintain in said cell culture medium a parameter selected from the group consisting of: concentration of lactate in said medium of not more than about 1.58 grams / liter; concentration of glucose in said medium of between about 0.5 and 1.0 grams / liter; and concentration of glucose in said medium of not less than about 2.89 grams / liter.

In a particular embodiment, the substrate provides at least about 60 m² of surface area for adherent cell culture, in particular at least about 400 m² of surface area for adherent cell culture. In yet another embodiment, the bioreactor is configured to contain at least about 20 liters of cell culture media.

It is also herein described an adherent cell culture bioreactor providing a substrate for adherent cell growth and configured to contain cell culture media and having a pump able to pump said media, the pump configured to be able to provide a constant-rate output of media at an output volume of less than about 50 mL media per m² substrate area per day. In a particular embodiment, the adherent cell culture bioreactor is configured to contain at least about 20 liters of cell culture media. In a further embodiment, the substrate provides at least about 60 m² of surface area for adherent cell culture, in particular at least about 400 m² of surface area for adherent cell culture. In yet another embodiment, the pump is connected to the substrate via a tube having an interior diameter sized to accommodate the pump output volume of fluid without the formation of air bubbles inside the tube.

The disclosure also provides a recombinant polypeptide produced by culturing adherent producer cells in the adherent cell bioreactor of the previous paragraph. In a particular embodiment, the polypeptide comprises virus. In another particular embodiment, the virus comprises a viral capsid containing a non-viral transgene.

It is also herein disclosed a method for culturing adherent cells on a substrate comprising:
a. obtaining the bioreactor described in the second to last paragraph, and then
b. adding to the bioreactor cell culture media and cells, and then
c. culturing the cells in adherent mode on the substrate, while circulating the cell culture media about the cells at a rate of less than about 50 mL of media per m² substrate area per day. In a particular embodiment, the substrate provides at least about 60 m² of surface area for adherent cell culture, in particular at least about 400 m² of surface area for adherent cell culture. In a further embodiment, the bioreactor is configured to contain at least about 20 liters of cell culture media.

The present disclosure also provides a method comprising:
a. obtaining an adherent cell culture bioreactor having a substrate for adherent cell culture and a first sampling tube having an input end and an output end, the input end of the first sampling tube in communication with the substrate whereby liquid can flow from the substrate into the input end of the first sampling tube, through the first sampling tube and exit the bioreactor at the output end of the first sampling tube; and then
b. adding cell culture media to the bioreactor, seeding cells into the bioreactor and culturing cells adherent to the substrate, and
c. flowing the cell culture media through the first sampling tube, and then
d. removing from the first sampling tube output end at least one removed portion of cell culture media; and then
e. permanently sealing the first sampling tube without returning into the output end of the sampling tube a material amount of the removed portion of cell culture media.

In a particular embodiment, the adherent cell culture bioreactor further comprises a second sampling tube having an input end and an output end, the input end in communication with the substrate whereby liquid can flow from the substrate into the input end of the second sampling tube, through the second sampling tube and exit the bioreactor at the output end of the sampling tube; the method further comprising:
f. flowing the cell culture media through the second sampling tube, and then
g. removing from the second sampling tube output end a second removed portion of cell culture media; and then
h. permanently sealing the second sampling tube without returning into the output end of the second sampling tube a material amount of the removed cell culture media.

In a particular embodiment, the bioreactor is configured to contain at least about 20 liters of cell culture media. In yet another embodiment, the substrate provides at least about 60 m² of surface area for adherent cell culture, in particular at least about 400 m² of surface area for adherent cell culture. In another embodiment, the cells adherent to the substrate express a recombinant polypeptide.

It is further disclosed the recombinant polypeptide produced by the method of the previous paragraph. in a particular embodiment, the polypeptide comprises virus. In another embodiment, the virus comprises a viral capsid containing a non-viral transgene.

The present disclosure also provides an adherent cell culture bioreactor comprising:
a. a substrate for adherent cell culture in flowable communication with a first pair of closable sampling tubes, whereby cell culture media can flow from the substrate through each sampling tube, and whereby each sampling tube may be aseptically closed after cell culture media flows from the substrate through the sampling tube, to prevent further media flow through the sampling tube; and
b. at least one of the pair of sampling tubes being configured to be aseptically attached to a detachable sampling container.

In a particular embodiment, the bioreactor is configured to contain at least about 20 liters of cell culture media. In another embodiment, the bioreactor further comprises a second pair of closable sampling tubes, at least one of the second pair of sampling tubes being configured to be aseptically attached to a detachable sampling container. In yet another embodiment, the bioreactor further comprises a third pair of closable sampling tubes, at least one of the third pair of sampling tubes being configured to be aseptically attached to a detachable sampling container. In another embodiment, the substrate provides at least about 60 m² of surface area for adherent cell culture, in particular at least about 400 m² of surface area for adherent cell culture.

It is also disclosed a recombinant polypeptide produced by the bioreactor described in the previous paragraph. In a particular embodiment, the polypeptide comprises virus. In a further embodiment, the virus comprises a viral capsid containing a non-viral transgene.

It is also further disclosed herein an adherent cell culture bioreactor vessel having a substrate for adherent cell culture and a pump able to provide a constant-rate output of cell culture media sufficient to exchange the cell culture media in the vessel less than once every about 3 ½ days. In a particular embodiment, the pump is able to provide a constant-rate output of cell culture media sufficient to exchange the cell culture media in the vessel less than once every about 4 ½ days. In a further particular embodiment, the substrate provides at least about 60 m² of surface area for adherent cell culture, in particular at least about 400 m² of surface area for adherent cell culture. In yet another embodiment, the adherent cell culture bioreactor is configured to contain at least about 20 liters of cell culture media.

It is also disclosed a recombinant polypeptide produced by culturing adherent producer cells in the adherent cell bioreactor described in the previous paragraph. In a particular embodiment, the polypeptide comprises virus. In another embodiment, the virus comprises a viral capsid containing a non-viral transgene.

The present disclosure also provides a method for culturing adherent cells on a substrate comprising:
a. obtaining the bioreactor described in the second to last paragraph, and then
b. adding to the bioreactor cell culture media and cells, and then
c. culturing the cells in adherent mode on the substrate, while circulating the cell culture media about the cells at a rate of less than about 50 mL of media per 1,800 cells per day.

In a particular embodiment, the substrate provides at least about 400 m² of surface area for adherent cell culture. In a further embodiment, the bioreactor is configured to contain at least about 20 liters of cell culture media.

### Brief Description Of the Drawings

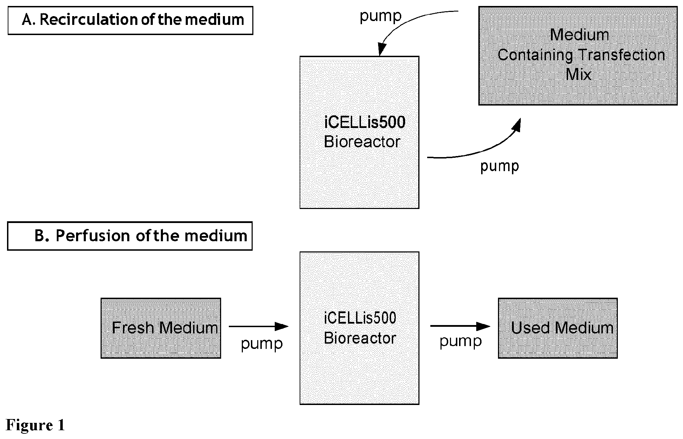

Figure 1 schematically illustrates two different perfusion methods.
Figure 2 shows a way to connect a bioreactor vessel to a sampling manifold.
Figure 3 shows a version of our improved sampling manifold.

### Detailed Description

We have implemented a way to improve medium feed in and feed out strategy in large capacity adherent cell culture bioreactors such as, for example, the iCELLis^{™} 500 bioreactor. Commercially-available large scale bioreactors are designed to *recirculate* media at *high velocity*; we found that *perfusing* media at a *low velocity* unexpectedly increases producer cell output. Our approach is the only suitable way to achieve acceptably slow and constant cell culture medium flow into and out of the bioreactor vessel. Constant medium flow supports high viral productivity for infected, transfected or transduced producer cells, while avoiding excessive medium usage.

Adherent cell culture bioreactor vessels provide a substrate on which cultured cells can adhere and grow. That substrate is housed in a container which contains liquid cell culture media.

Commercially-available large capacity adherent cell culture bioreactors are provided with "Feed In" and "Feed Out" pumps. These pumps respectively pump cell culture media into and out of the container which contains the substrate, thus providing the cultured cells with fresh culture media, and removing spent media and its appurtenant culture waste products.

### Example 1 - Intermittent Pumping

Pumps can be programmed to run constantly. Run constantly at minimum speed, the prior art iCELLis^{™} 500 Feed In and Feed Out pumps produce at least about 42.3 L of medium flow per day (running at the slowest rate possible, 24 rpm). The capacity of the bioreactor vessel being about 25 liters, the medium in the reactor is completely exchanged almost twice a day.

We used a commercially-available large capacity adherent cell culture bioreactor vessel to culture transfected "producer" cells which express viral polypeptides and produce viral particles. We found that these producer cells are most productive when the cell culture medium is perfused at a rate slower than the slowest pump output volume available in commercially-available apparatus. To be able to get a properly low medium perfusion rate for our purposes, we programmed the pumps to run for certain time interval, followed by an interval where the pumps did not pump at all.

Because of this need to vary pump output, we have seen in practice that the stock iCELLis^{™} 500 bioreactor Feed Out pump provided by the manufacturer is not capable of removing medium out from the bioreactor vessel in a well controlled manner. We thus becan to test the viability of lower-output pumps able to provide an adequately low output flow.

### Example 2 - Constant Low-Velocity Pumping

We then investigated whether the variable media flow used in the prior art might impact cell culture in some way. To do this, we replaced the prior art Feed In pump provided with the iCELLis^{™} 500 bioreactor with a replacement pump which was able to provide a lower output volume, about 16.7 L/day. For a 25 liter capacity bioreactor vessel, this means the media in the vessel would be exchanged once every 3 ½ to 4 ½ days, rather than the nearly twice a day typical in the prior art. This reduces the flow across *e.g.*, a 100m² substrate surface area from at least 42.3 L per 100 m² per day to 16.7 L per 100 m² per day. This lower Feed In pump rate enabled us to for the first time to run the Feed In pump constantly, without periodic stoppages.

During these runs it was observed that the Feed Out pump provided by the manufacturer was not able to perform the removal of the media from the bioreactor vessel throughout the process as planned. We therefore similarly replaced the prior art Feed Out pump provided by the manufacturer with a lower-output pump.

We performed a commercial-scale manufacturing run, using recombinant adherent producer cells to produce a recombinant adenovirus bearing a transgene (useful for *e.g.*, gene therapy), using in the adherent cell culture process a combination of the stock iCELLis^{™} 500 Feed In pump and our own lower-output Feed Out pump. We have used several transgenes (e.g., coding for interferon, or for herpes simplex thymidine kinase). Our method is generally advantageous in producing vector with any kind of transgene (including therapeutic transgenes and marker transgenes such as green fluorescent protein), or any other genetic element or nucleotide sequence (e.g., viral vector containing RNA transgene, shRNA, IngRNA, eRNA etc.).

We also performed three commercial-scale manufacturing runs, wherein we used our own lower-output Feed Out pump and also replaced the stock iCELLis^{™} 500 high-output Feed In pump also with a lower-output pump. We received significantly higher adenoviral production in the adherent producer cells in each of these three runs. In these three runs, the productivity of viral particles per cell had increased 49.4 % as compared to productivity using the prior art higher-output pumps.

When we changed the process to work with lower-output pumps, our viral productivity per cell surprisingly increased 49.4%. Without intending for the legal coverage of our patent to be bound by any scientific causal theory, this improvement may be due to feeding the bioreactor vessel constantly with fresh medium, thereby keeping stable the level of nutrients in the media in contact with the cultured cells. For example, we have found that adherent cells are most productive when the media flow is substantially constant and slow enough to maintain in the cell culture medium a level of lactate of not more than about 1.6 grams lactate / liter of culture medium. Similarly, we have found that certain adherent producer cells are most productive when the concentration of glucose in the culture media is maintained at between about 0.5 and about 1.0 grams of glucose per liter of media. Other adherent producer cells are most productive with a glucose concentration which is higher (*e.g.,* at least about 2.9 grams per liter) but nonetheless maintained at a relatively constant concentration due to low-velocity but substantially constant media flow. Alternatively, this may be due to the slow but constant flushing away of unwanted cellular waste products from the cell surface. Alternatively, this could be due to avoiding the physical shear stress placed on the cultured cells when using the higher-velocity media flow required by prior art hardware. Alternatively, this increase could be due to slower media flow enabling each producer cell a longer time to produce virus-like particles. Whatever the cause (or causes), we found that a slower, constant medium flow surprisingly and significantly increased adherent producer cell productivity.

This increase is particularly surprising in light of the fact that suspension cell culture (*e.g.*, the CultiBag RM^{™} suspension cell culture bag, commercially available from Sartorius Corp., Cambridge Massachusetts) provides more-or-less constant media flow across the cell surface, yet suspension cell culture is known in the art as less productive than is adherent cell culture.

Lower-output pumps may also be successfully used for inoculation of the host cells into the adherent bioreactor vessel, for drawing samples of the cell culture media during cell growth, and for harvesting e.g., culture media at the end of the cell growth. We have surprisingly found that using a lower-output pump to perform these functions counter-intuitively makes each of those processes faster than with the prior art high-output pumps.

One may replicate our system by simply obtaining a commercially-available large-scale adherent cell culture bioreactor vessel, disconnecting the Feed In and Feed Out tubing from the pumps provided with that bioreactor, and connecting that tubing to external pumps. With external pumps, however, the operator may lose the ability to control the external pumps through the computer which controls the bioreactor (and its integral pumps). For example, commercially available large scale adherent bioreactors typically include a sensor which measures the level of liquid cell culture medium in the bioreactor vessel, to monitor whether the cell culture substrate is adequately submerged in medium. External pumps may not be automatically controlled by the feedback control loops that are automatically activated by the liquid cell culture medium level sensed in the bioreactor vessel. Similarly, commercially available large scale adherent bioreactors typically include a recording device which records the running speeds of the pumps. Using external pumps, the output may not be recorded automatically to the computer. One may overcome these disadvantages by calibrating the external pumps before use and manually monitoring the culture medium flow in and out from the bioreactor vessel regularly, and by recording all steps manually to the batch manufacturing records. Alternatively, one can connect the new pumps to the bioreactor control computer, so that the low output pumps can be controlled and monitored similarly and simultaneously, as for the bioreactor itself.

The pump(s) can be controlled by software or through external calibration, or by controlling by nominal motor speed (RPMs), or using a balance or weight-controlled system. Alternatively, the pump(s) may be controlled manually, or have an automatic speed control or any other suitable way to control the pump. The precise control method is not critical, as long as the pump is able to provide the consistent low-flow output we have surprisingly found is favorable to increased adherent cell production.

Our lower flow rate is effective for larger-area adherent cell cassettes (e.g., a 500 m² cassette). Lower flow rate may also be effectively used with smaller-area substrate cassettes, such as 66 m² fiber cassettes.

Our system can be used to culture adherent producer cells which express viral polypeptide and thus produce recombinant virus or virus-like particles (we use the term "virus" in our appended legal claims to encompass both viruses and virus-like particles).

### Example 3 - Unidirectional Sampling Manifold

A bioreactor sampling manifold is used for taking liquid samples of the cell culture medium from the bioreactor vessel to e.g., check pH, or to measure glucose, metabolites and cell waste products (e.g., lactate) or other substances in the medium.

We unexpectedly found that the stock sampling manifold offered as part of the commercially-available iCELLis^{™} is vulnerable to contamination. We have successfully designed a new sampling manifold for the bioreactor for removing the need of pumping medium in and out from the bioreactor vessel into a sampling bottle. This way we minimize the risk of contamination due to a sampling.

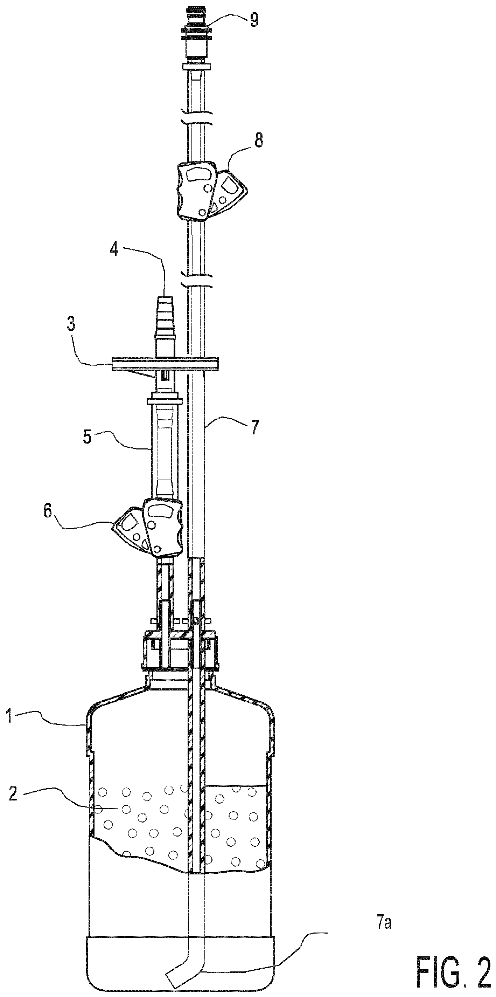

A schematic drawing of an adherent culture bioreactor is shown in Figure 2. The bioreactor is comprised of a vessel [1] which contains a cell culture substrate [2] to which cells adhere when cultured in adherent mode. The vessel [1] is filled with liquid cell culture medium which irrigates the cell culture substrate [2]. The vessel [1] has a vent which includes an air filter [3] having an exit vent [4]. The exit vent [4] is able to vent excess gas out of the bioreactor vessel and the filter [3] filters the gas flowing through the filter to prevent particulate contaminants (e.g., viral particles, fungal spores) from exiting or entering the bioreactor vessel. The filter [3] is preferably detachably connected to the vessel [1] via a vent tube [5]. Alternatively, the filter may be integrated into the body of the vessel [1], albeit this risks enabling liquid cell culture media to splash onto the filter and occlude it. Preferrably, the vent tube has a clamp [6] which enables a technician to close the vent tube [5] as and when desired.

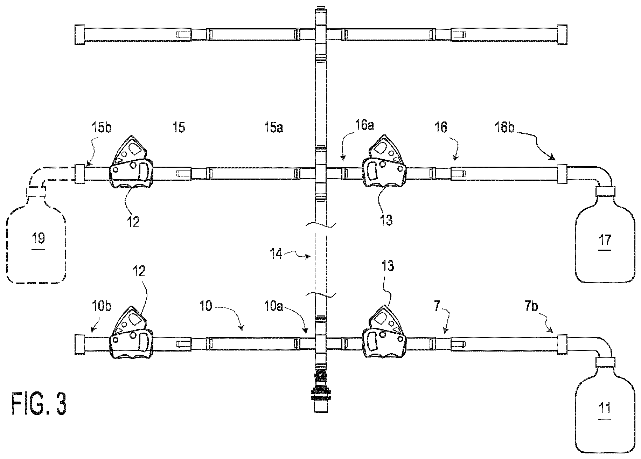

The vessel [1] also has a sampling tube [7] with an input end [7a] which passes into the liquid cell culture medium and thus is able to intake liquid medium. Preferably, the sampling tube [7] also has a clamp [8] which enables a technician to close the sampling tube [7] as as when desired. The sampling tube [7] has a connection [9] enabling it to be connected to a sampling device. The sampling device may be a detachable sampling bag or bottle. We prefer, however, the sampling device comprise a sampling manifold [Figure 3] able to connect to a pair of detachable sampling bags or bottles.

The sampling manifold [Figure 3] is connected [9] to the sampling tube [7]. The sampling manifold comprises a waste tube [10] having an input end [10a] and an output end [10b], and the sampling tube [7] output end [7b] able to connect to a removable sampling container [11]. The sampling container [11] may be, for example, a bag or a bottle.

The prior art commercially-available sampling manifold is based on the idea that first approximately 200 ml of cell culture media is merely a rinse; it is pumped through the manifold to rinse the interior of the manifold. That rinse media passes out of the manifold through an exit opening into a temporary storage bottle. Additional cell culture media is then pumped through the manifold, and a sample is collected. After the sample is taken, the approximately 200 ml of cell culture rinse media which was initially collected in the temporary storage bottle is pumped from the bottle back into the manifold and back into the bioreactor vessel [1], so that as little as media as possible remains inside the sampling manifold. The temporary storage bottle and the tubing can be emptied because the prior art configuration provides an aseptic filter in the sampling bottle, which filter permits ambient air to exit and enter the bottle (when liquid is pumped into and out of the bottle, respectively), the filter filtering airborne contaminants in the ambient air and thus preventing contamination of the cell culture medium.

If the integrity of the filter is compromised, however, this can pose a major contamination risk which is difficult to detect until an entire manufacturing batch has been contaminated. Using the commercially-available prior art configuration, we have in fact incurred a contamination of a commercial-scale manufacturing batch. In evaluating the failure, it was observed that the aseptic air filter in a sampling bottle was broken, but it remained unclear how it had broken.

We thus designed a new sampling manifold using a different sampling technique. We have tested it successfully in at least four commercial-scale manufacturing batches, and have encountered no problems.

With our sampling manifold we eliminate the need to pump cell culture media liquid out from the bioreactor vessel and then back again. Rather, with our sampling manifold, when liquid is taken from the bioreactor vessel, it is never pumped back in again. This reduces the risk of contamination significantly.

Our sampling manifold is described in Figure 3. We prefer to make our sampling manifold from commercially-available C-flex tubing, Masterflex^{™} tubing, clamps, and commercially-available polymer tube connectors. The tubing can be non-sterile or sterilized by e.g., temperature- or moisture-based techniques such as autoclaving, or gamma-irradiated or gas-based sterilization such as ethylene. The tubing can be ready-made, or custom-made by the user. We merely prefer it to be compatible with commercially-available sterile connections, thus able to be branched with any type connections, and suitable for sealing and welding.

Our sampling manifold can be connected to a bioreactor such as the iCELLis^{™} 500 bioreactor through MPC-connector. When used with an iCELLis bioreactor, our sampling manifold can entirely replace the sampling manifold offered by Pall Life Sciences as part of their commercially available Starter Kit, which is marketed together with the iCELLis^{™} 500 bioreactor.

In our sampling manifold, we use a pair of tubes to take one sample. One tube is used to rinse the manifold interior, the other tube is used after rinsing to take the sample. For example, one can use tube [15] to rinse the manifold and then use tube [7] to take a sample.

To begin, a single-use sampling bag or bottle [11, 17, 19] is aseptically attached (we prefer through welding) to the sampling tube [7]. One may (and we prefer) to also attach a sampling bag [19] to the first (rinse) line of the pair [15]. Alternatively, the first line may drain into a waste bottle etc.

To use our sampling manifold, liquid cell culture media is pumped from the bioreactor vessel [1] or an accompanying media reservoir through the manifold to rinse the interior . This is for rinsing the manifold and getting fresh medium/liquid from the vessel [1] into the manifold. This rinse media is removed from the manifold via a rinse tube [15] into a rinse media container [19]. Eherinse tube [15] is then sealed or closed. This may be done with a tubing clamp [12] (as illustrated). Alternatively, the tubing may be sealed with an end cap [on 15b], or welded closed [at 15b], etc.: the manner of sealing is not particularly important here. The rinse tube [15] thereafter remains sealed off for the remainder of the manufacturing run. In the appended legal claims, we refer to sealing off for the remainder of the manufacturing run as "permanent" sealing because in the context of a given manufacturing run, it is effectively permanent (*i.e*., lasting to the end).

The sampling tube exit end [7b], going to the sample container [11], is then opened [13]. Fresh medium/liquid is pumped from the bioreactor vessel [1] (*e.g.*, from the part of the bioreactor which houses the adherent cell culture substrate) into the sampling tube [7], and then out of it [7b] into the sampling container [11]. While we illustrate one sample container ([11]), one could alternatively use several sample containers and take several physically-separate samples. The sampling tube [7] is then closed [13]. The sampling container [11] may then be sealed and removed from the sampling tube output [7b]. After taking the sample, the pair of manifold branches [15, 7] remain sealed off for the remaining duration of the manufacturing run. We prefer both tubes be drained to remove residual media, to reduce risk of contamination.

Sampling bags [11] provide a sampling port used for aseptically drawing liquid out of the sampling bag to assay. Several samples can thus be taken from one bag aseptically.

To enable sequential sampling at different times, we prefer to provide several pair of sampling tubes. With this, a second (or subsequent) sampling may be performed in a similar fashion as the first one, using a different pair of manifold tubes and a second sampling bag attached to the second tube of that second pair of tubes. For example, to take a second sample, one may use line [10] as the rinse tube and line [16] as the sampling tube, with the sample being taken in container [17]). Media/liquid that has remained in the manifold since the first sampling is discarded by pumping it out of the manifold [10b], preferrably into a rinse or waste receptacle or container until fresh media/liquid from the bioreactor substrate has filled the manifold. This second rinse tube [10] is then sealed or closed. This may be done with a tubing clamp [12] (as illustrated). Alternatively, the tubing may be sealed with an end cap, or welded closed, etc.: the manner of sealing is not particularly important here. The second rinse tube [10] thereafter remains sealed off for the remainder of the manufacturing run.

The second sampling tube [16] is then opened [13]. Fresh medium/liquid is pumped from the bioreactor vessel [1] (*e.g.*, from the part of the bioreactor which houses the adherent cell culture substrate) into the second sampling tube [16] and then out of it [16b] into a second sampling container(s) [17]. The the sampling tube of the second pair [16] is then closed [13]. The sampling container(s) [17] may then be sealed and removed. After taking the sample, the pair of manifold branches [10, 16] remain sealed off for the remaining duration of the manufacturing run.

This configuration may be repeated as desired, providing as many pair of rinse and sampel tubes as desired to enable as many different sampling times as desired.
With our sampling port, a disadvantage at the moment is the need to construct the manifold (rather than be able to purchase it commercially). Another disadvantage could be the use of several sampling bags for each manufacturing batch. These disadvantages, however, are more than off-set by the reduced possibility of contaminating a manufacturing batch, which in commercial-scale manufacturing poses a significant financial risk.

## Claims

1. An adherent cell culture bioreactor comprising:
a substrate for adherent cell growth, wherein the substrate has a surface area; and
a pump, wherein the pump is configured to provide a constant-rate output of cell culture media at an output volume of less than 50 ml media per m² surface area of the substrate per day, and wherein the bioreactor is configured to contain at least 20 liters of cell culture media.

2. The adherent cell culture bioreactor of claim 1, wherein the substrate comprises at least 60 m² of surface area.

3. The adherent cell culture bioreactor of claim 2, wherein the substrate comprises at least 400 m² of surface area.

4. The adherent cell culture bioreactor of claim 1, wherein the pump is connected to the bioreactor via a tube having an interior diameter sized to accommodate the pump output volume of fluid without the formation of air bubbles inside the tube.

5. A method for culturing adherent cells on a substrate comprising:
a. obtaining the bioreactor of claim 1, and then
b. adding to the bioreactor cell culture media and cells, and then
c. culturing the cells in adherent mode on the substrate, while circulating the cell culture media about the cells at a rate of less than about 50 ml of media per m² surface area of the substrate per day.

6. The method of claim 5, wherein the cells in adherent mode produce a recombinant polypeptide.

7. The method of claim 6, where the cells in adherent mode produce a virus.

8. The method of claim 7, where the virus comprises a viral capsid containing a non-viral transgene.

9. The method of claim 5, wherein the substrate comprises at least 60 m² of surface area.

10. The method of claim 9, wherein the substrate comprises at least 400 m² of surface area.

## Patentansprüche

1. Ein adhärenter Zellkultur-Bioreaktor umfassend:
ein Substrat für adhärentes Zellwachstum, wobei das Substrat eine Oberfläche hat; und eine Pumpe, wobei die Pumpe konfiguriert ist um eine konstante Ausstoßrate des Zellkulturmediums bei einem Ausstoßvolumen von weniger al 50 ml Medium pro m² Oberfläche des Substrats pro Tag zu ermöglichen und wobei der Bioreaktor konfiguriert ist, um mindestes 20 Liter des Zellkulturmediums zu enthalten.

2. Der adhärente Zellkultur-Bioreaktor nach Anspruch 1, wobei das Substrat mindestens 60 m² der Oberfläche umfasst.

3. Der adhärente Zellkultur-Bioreaktor nach Anspruch 2, wobei das Substrat mindestens 400 m² der Oberfläche umfasst.

4. Der adhärente Zellkultur-Bioreaktor nach Anspruch 1, wobei die Pumpe mit dem Bioreaktor über einen Schlauch mit einem Innendurchmesser verbunden ist, der so bemessen ist, dass er das Fördervolumen der Pumpe aufnehmen kann, ohne dass sich Luftblasen im Inneren des Schlauches bilden.

5. Ein Verfahren zur Kultivierung von adhärenten Zellen auf einem Substrat umfassend:
a. Erhalten des Bioreaktors aus Anspruch 1 und dann
b. Zugeben von Zellkulturmedium und Zellen zum Bioreaktor und dann
c. Kultivieren der Zellen in adhärentem Modus auf dem Substrat, während das Zellkulturmedium mit einer Geschwindigkeit von weniger als etwa 50 ml Medium pro m² Oberfläche des Substrats pro Tag um die Zellen zirkuliert.

6. Das Verfahren nach Anspruch 5, wobei die Zellen im adhärenten Modus ein rekombinantes Polypeptid produzieren.

7. Das Verfahren nach Anspruch 6, wobei die Zellen im adhärenten Modus ein Virus produzieren.

8. Das Verfahren nach Anspruch 7, wobei das Virus ein virales Kapsid enthaltend ein nicht-virales Transgen umfasst.

9. Das Verfahren nach Anspruch 5, wobei das Substrat mindestens 60 m² der Oberfläche umfasst.

10. Das Verfahren nach Anspruch 9, wobei das Substrat mindestens 400 m² der Oberfläche umfasst.

## Revendications

1. Bioréacteur de culture de cellules adhérentes comprenant :
un substrat pour la croissance de cellules adhérentes, dans lequel le substrat a une aire surfacique ; et
une pompe, dans lequel la pompe est conçue pour produire un débit constant de milieu de culture cellulaire à un volume de sortie inférieur à 50 ml de milieu par m² d'aire surfacique du substrat par jour, et dans lequel le bioréacteur est configuré pour contenir au moins 20 litres de milieu de culture cellulaire.

2. Bioréacteur de culture de cellules adhérentes selon la revendication 1, dans lequel le substrat comprend au moins 60 m² d'aire surfacique.

3. Bioréacteur de culture de cellules adhérentes selon la revendication 2, dans lequel le substrat comprend au moins 400 m² d'aire surfacique.

4. Bioréacteur de culture de cellules adhérentes selon la revendication 1, dans lequel la pompe est reliée au bioréacteur par le biais d'un tube ayant un diamètre intérieur dimensionné pour accueillir le volume de fluide de sortie de pompe sans formation de bulles d'air à l'intérieur du tube.

5. Méthode de culture de cellules adhérentes sur un substrat comprenant :
a. l'obtention du bioréacteur selon la revendication 1, et puis
b. l'ajout au bioréacteur du milieu de culture cellulaire et des cellules, et puis
c. la culture des cellules en mode adhérent sur le substrat, tout en faisant circuler le milieu de culture cellulaire autour des cellules à un taux inférieur à environ 50 ml de milieu par m² d'aire surfacique du substrat par jour.

6. Méthode selon la revendication 5, dans laquelle les cellules en mode adhérent produisent un polypeptide recombinant.

7. Méthode selon la revendication 6, où les cellules en mode adhérent produisent un virus.

8. Méthode selon la revendication 7, où le virus comprend une capside virale contenant un transgène non viral.

9. Méthode selon la revendication 5, dans laquelle le substrat comprend au moins 60 m² d'aire surfacique.

10. Méthode selon la revendication 9, dans laquelle le substrat comprend au moins 400 m² d'aire surfacique.
